# FASCICULE DE BREVET EUROPEEN

(11) **EP 0 559 264 B1**
(45) Date de publication et mention de la délivrance du brevet: **02.05.1997**
(21) Numéro de dépôt: 93200456.7
(22) Date de dépôt: 18.02.1993
(51) Int. Cl.: C07C 17/42, C07C 19/08

(54) **Procédé de stabilisation d'un hydrofluoroalcane et compositions comprenant au moins un hydrofluoroalcane**
Verfahren zur Stabilisierung eines Fluorkohlenwasserstoffs und Mischungen, die mindestens einen Fluorkohlenwasserstoff enthalten
Process for the stabilization of a hydrofluoroalkane and compositions containing at least a hydrofluoroalkane

(30) Priorité: 02.03.1992 BE 9200206
(43) Date de publication de la demande: 08.09.1993
(73) Titulaire: SOLVAY (Société Anonyme), B-1050 Bruxelles (BE)
(72) Inventeur: Paulus, Mireille, B-1020 Bruxelles (BE); Barthelemy, Pierre, B-1315 Pietrebais (BE)
(74) Mandataire: Jacques, Philippe

(56) Documents cités:
- DE-C- 562 820
- CHEMICAL ABSTRACTS, vol. 77, no. 13, 25 Septembre 1972, Columbus, Ohio, US; abstract no. 87835n, OSAMU IKEDA ET AL. 'Stabilization of halogenated hydrocarbons' page 400 ;colonne 1 ;
- PATENT ABSTRACTS OF JAPAN vol. 14, no. 417 (C-756)(4360) 10 Septembre 1990
- PATENT ABSTRACTS OF JAPAN vol. 15, no. 38 (C-800)30 Janvier 1991

## Description

La présente invention se rapporte à la stabilisation des hydrofluoroalcanes, et plus particulièrement à la stabilisation du 1-1-dichloro-1-fluoroéthane.

Suspectés d'attaquer la couche d'ozone stratosphérique, les chlorofluorocarbures entièrement halogénés (CFC) sont progressivement remplacés par divers substituts, notamment par des fluorocarbures et chlorofluorocarbures partiellement halogénés, généralement désignés sous le terme générique hydrofluoroalcanes (HFA). Le 1-1-dichloro-1-fluoroéthane (HFA-141b), par exemple, est un hydrofluoroalcane qui s'avère être un substitut intéressant de certains CFC, notamment comme agent gonflant de mousses polymériques ou comme solvant.

Il est généralement reconnu que les hydrofluoroalcanes doivent être stabilisés pour éviter tout risque de dégradation en cours de stockage ou d'utilisation, plus particulièrement lorsqu'ils sont utilisés comme solvants. Une dégradation potentielle de ces composés est liée à leur possible hydrolyse, provoquant la formation d'HCl. Lorsqu'ils sont utilisés comme solvant de dégraissage de pièces métalliques, le risque de dégradation est accru en raison de la possible action catalytique de ces métaux sur la réaction d'hydrolyse.

La demande de brevet examinée JP-B-72/22562 de SHOWA-DENKO enseigne, dans son principe général, la stabilisation d'hydrocarbures bromochlorofluorés, utilisés comme anesthésiants, par addition de 0,01 à 1 % en poids d'un alcanol inférieur.

La demande de brevet non examinée JP-A-02/273632 de ASAHI divulgue des compositions azéotropiques ou pseudo-azéotropiques renfermant de 99,9 à 90 % de 1-1-dichloro-1-fluoroéthane et de 0,1 à 10 % de méthanol ou d'éthanol.

Dans le brevet US-A-4,842,764 de ALLIED-SIGNAL on enseigne cependant qu'une composition solvante à base de chlorofluoro-carbure et d'un alcanol inférieur est très agressive vis-à-vis de certaines surfaces métalliques.

La demande de brevet non examinée JP-A-01/132539 de ASAHI enseigne un procédé de stabilisation thermique de compositions solvantes azéotropiques renfermant 73,5 % de 1,2-dichloro-1-fluoroéthane et 26,5 % de méthanol par addition de divers stabilisants.

Le brevet DE-562820 décrit la stabilisation de certains hydrocarbures chlorés par addition de 0,0001 à 0,1 % en poids d'amylène.

La demande de brevet WO 91/18852 de Solvay enseigne qu'en présence d'au moins 10 ppm d'acides de Lewis, le 1,1-dichloro-1-fluoroéthane peut subir une dégradation. Or, il a maintenant été observé que même de plus faibles quantités d'acides de Lewis, en particulier de type halogénures métalliques, peuvent induire une dégradation importante des hydrofluoroalcanes tel le 1,1-dichloro-1-fluoroéthane. Ainsi, de 2 à 3 ppm de chlorure de fer dissous dans le 1-1-dichloro-1-fluoroéthane suffisent pour altérer très rapidement la qualité du produit de façon inacceptable, même à température ambiante. En outre, il a encore été observé que les hydrofluoroalcanes renfermant des traces d'acides de Lewis de type halogénures métalliques s'avèrent très agressifs à l'encontre de surfaces métalliques.

L'invention vise à remédier aux inconvénients décrits plus haut, en fournissant un procédé de stabilisation d'hydrofluoroalcanes, qui évite notamment une dégradation de ceux-ci par les acides de Lewis de type halogénures métalliques. Elle vise aussi à fournir, en tant que produit nouveau, l'hydrofluoroalcane ainsi stabilisé, non agressif à l'encontre des surfaces métalliques.

La présente invention a dès lors pour objet un procédé de stabilisation d'un hydrofluoroalcane à l'encontre de la dégradation provoquée par les acides de Lewis de type halogénures métalliques, selon lequel on ajoute audit hydrofluoroalcane au moins un alcool et/ou au moins un hydrocarbure insaturé à titre de stabilisant, qui se caractérise en ce qu'on sélectionne l'alcool parmi le méthanol, l'éthanol et l'éthylène glycol et l'hydrocarbure insaturé parmi les alcènes en C5-C6, et en ce qu'on met en oeuvre ledit alcool en une quantité pondérale au moins égale à environ 0,001 % et inférieure à 0,1 % du poids de l'hydrofluoroalcane et ledit hydrocarbure insaturé en une quantité au moins égale à environ 0,001 % et inférieure à environ 0,5 % du poids de l'hydrofluoroalcane.

Par hydrofluoroalcane, on entend généralement désigner tout hydrocarbure halogéné saturé, de type acyclique ou alicyclique, comprenant au moins un atome d'hydrogène et au moins un atome de fluor. Ces hydrofluoroalcanes peuvent ou non comprendre en outre un ou plusieurs atomes de chlore ou de brome. De préférence, ils ne comprennent pas de brome.

Les hydrofluoroalcanes tels que définis comprennent généralement de 1 à 6 atomes de carbone.

Par acides de Lewis de type halogénures métalliques, on entend désigner des halogénures métalliques qui possèdent les propriétés des acides de Lewis, c'est-à-dire dont la structure électronique est telle qu'ils sont capables d'accepter des électrons de réactifs basiques. De tels acides de Lewis sont notamment FeCl₃, AlCl₃, SbCl₅, SbCl₃, SnCl₄, TiCl₄, BCl₃ et BF₃.

On a observé, de manière très surprenante, que l'addition en quantité inférieure à 0,1 % d'un alcool choisi parmi le méthanol, l'éthanol et l'éthylène glycol à un hydrofluoroalcane inhibe toute dégradation du produit en présence d'acides de Lewis de type halogénures métalliques, sans rendre ce produit agressif à l'encontre de surfaces métalliques, alors que l'on a observé, par ailleurs, que d'autres alcools, comme l'isopropanol, même utilisés en quantités élevées, supérieures à 0,1 % du poids de l'hydrofluoroalcane, sont impuissants à inhiber la dégradation des hydrofluoroalcanes par les acides de Lewis de type halogénures métalliques.

Le méthanol et l'éthylène glycol sont préférés.

Le méthanol est l'alcool tout particulièrement préféré.

De manière générale, dans le procédé selon l'invention, on ajoute l'alcool en une quantité au moins égale à 0,001 % et inférieure à 0,1 % du poids de l'hydrofluoroalcane à stabiliser.

Des quantités spécialement recommandées pour l'alcool sont celles supérieures ou égales à 0,005 % du poids de l'hydrofluoroalcane, de préférence supérieures ou égales à 0,008 % et celles inférieures ou égales à 0,09 % du poids de l'hydrofluoroalcane, de préférence inférieures ou égales à 0,08 %.

Les hydrocarbures insaturés utilisables dans le procédé selon l'invention sont les alcènes en C5-C6. Par alcènes, on entend désigner les alcènes aliphatiques et les cycloalcènes. A titre d'exemples, on peut citer les méthylbutènes, les diméthylbutènes, les pentènes, les méthylpentènes, les hexènes, le cyclopentène, le cyclohexène et le méthylcyclopentène. Des hydrocarbures insaturés spécialement avantageux sont notamment le 2-méthylbut-2-ène, le 2-méthylbut-1-ène, le 3-méthylbut-1-ène, le 2-éthylbut-1-ène, le pent-1-ène, le pent-2-ène, le 2-méthylpent-1-ène, le 2-méthylpent-2-ène, le 3-méthylpent-1-ène, le 3-méthylpent-2-ène, le 4-méthylpent-1-ène, le hex-1-ène, le hex-2-ène et le hex-3-ène. Parmi ces hydrocarbures insaturés, le 2-méthylbut-2-ène, le 2-méthylbut-1-ène, le 3-méthylbut-1-ène et leurs mélanges sont préférés. De très bons résultats ont été obtenus avec du 2-méthylbut-2-ène industriel, habituellement dénommé amylène, lequel peut renfermer un peu de 2-méthylbut-1-ène.

De manière générale, dans le procédé selon l'invention, on ajoute l'hydrocarbure insaturé en une quantité au moins égale à 0,001 % et inférieure à 0,5 % du poids de l'hydrofluoroalcane à stabiliser.

De manière préférée, on ajoute le ou les hydrocarbures insaturés en une quantité d'environ 0,005 à environ 0,25 % du poids d'hydrofluoroalcane.

Dans un mode préféré de mise en oeuvre du procédé selon l'invention, on ajoute à l'hydrofluoroalcane à stabiliser, conjointement au moins un alcool et au moins un hydrocarbure insaturé.

De manière inattendue, l'addition conjointe à un hydrofluoroalcane d'au moins un alcool choisi parmi le méthanol, l'éthanol et l'éthylène glycol et d'au moins un hydrocarbure insaturé choisi parmi les alcènes en C5-C6, dans les quantités sélectionnées définies plus haut, stabilise l'hydrofluoroalcane de manière particulièrement efficace. L'existence d'une synergie entre l'alcool et l'hydrocarbure insaturé permet d'obtenir de très bons résultats de stabilisation par l'ajout d'environ 0,001 % à environ 0,075 % d'alcools, de préférence de méthanol, et d'environ 0,001 % à environ 0,5 % d'hydrocarbures insaturés, de préférence d'amylène. A titre illustratif, une très bonne inhibition de la dégradation du 1-1-dichloro-1-fluoroéthane en présence d'acides de Lewis de type halogénures métalliques peut être obtenue en ajoutant du méthanol en une quantité d'environ 0,005 % à environ 0,06 % du poids de 1-1-dichloro-1-fluoroéthane et de l'amylène en une quantité d'environ 0,005 % à environ 0,25 % du poids de 1-1-dichloro-1-fluoroéthane, la présence simultanée de méthanol et d'amylène provoquant un effet synergique sur la stabilité du 1-1-dichloro-1-fluoroéthane.

Le procédé selon l'invention est applicable pour stabiliser tout hydrofluoroalcane susceptible d'être en contact avec des acides de Lewis de type halogénures métalliques, soit durant le stockage, effectué généralement sous forme liquide, soit pendant sa mise en oeuvre ultérieure, sous forme liquide ou gazeuse.

Le procédé selon l'invention trouve une application intéressante pour la stabilisation des hydrofluoroalcanes acycliques ou alicycliques de formule générale CₐH_{b}F_{c}Cl_{d} dans laquelle a est un nombre entier de 1 à 6, b est un nombre entier de 1 à 13, c est un nombre entier de 1 à 13 et d est un nombre entier de 0 à 8, avec b + c + d = 2a + 2 lorsque le composé est acyclique et avec b + c + d = 2a lorsque le composé est alicyclique. Le procédé est particulièrement applicable aux composés acycliques répondant à la formule générale ci-dessus dans laquelle a est un nombre entier égal à 1 à 4, b est un nombre entier de 1 à 9, c est un nombre entier de 1 à 9 et d est un nombre entier de 0 à 5. Le procédé est tout particulièrement applicable aux composés acycliques répondant à la formule générale ci-dessus dans laquelle a est un nombre entier égal à 2 ou 3, b est un nombre entier de 1 à 6, c est un nombre entier de 1 à 6 et d est un nombre entier de 1 à 4. Des hydrofluoroalcanes pouvant être traités par le procédé de l'invention sont notamment les composés de formule brute CHClF₂, CH₂F₂, CH₃CCl₂F, CH₃CClF₂, CH₃CHF₂, CH₃CF₃, CH₂FCH₂F, CH₂FCHF₂, CH₂FCF₃, CHF₂CCl₃, CHF₂CF₃, CHCl₂CF₃, CHF₂CHF₂, CF₃CHClF, CF₃CF₂CHCl₂, CF₂ClCF₂CHClF, CF₃CH₂CF₂CH₃ et CF₃CH₂CH₂CF₃.

Le procédé selon l'invention donne de très bons résultats lorsqu'il il est appliqué à la stabilisation du 1-1-dichloro-1-fluoroéthane. Dans ce cas, d'excellents résultats sont obtenus, notamment en présence d'acides de Lewis de type halogénures métalliques, que ceux-ci soient présents en quantités infimes, par exemple de l'ordre de 0,1 mg par kilo d'hydrofluoroalcane, ou en quantités plus importantes, par exemple de l'ordre de plusieurs centaines de mg par kilo d'hydrofluoroalcane. Le procédé selon l'invention donne d'excellents résultats lorsque les compositions à base de 1-1-dichloro-1-fluoroéthane sont en présence d'une quantité d'acides de Lewis de type halogénures métalliques comprise entre environ 0,5 et 500 mg par kilo d'hydrofluoroalcane. De très bons résultats sont aussi obtenus avec le procédé selon l'invention lorsque la composition à base de 1-1-dichloro-1-fluoroéthane est en présence de quantités importantes d'acides de Lewis de type halogénures métalliques, de l'ordre d'un gramme ou plus d'acides de Lewis par kilo de 1-1-dichloro-1-fluoroéthane.

Les hydrofluoroalcanes stabilisés au moyen du procédé selon l'invention peuvent être stockés plusieurs mois, sans précautions particulières, sans qu'ils ne se dégradent. Leur comportement n'est nullement affecté par la présence de l'alcool ou de l'hydrocarbure insaturé.

Les hydrofluoroalcanes stabilisés au moyen du procédé selon l'invention conviennent pour toutes les utilisations classiques des hydrofluoroalcanes, par exemple comme solvant de nettoyage, comme agent gonflant de mousses polymériques ou comme fluide réfrigérant, sans qu'il soit nécessaire d'en éliminer toute trace d'acides de Lewis de type halogénures métalliques, ni de prendre des précautions lourdes et coûteuses afin d'éviter l'apparition de tels composés dans les réservoirs de stockage ou dans les nombreux dispositifs de mise en oeuvre.

L'invention concerne dès lors aussi des compositions stabilisées à l'encontre de la dégradation provoquée par les acides de Lewis de type halogénures métalliques, comprenant au moins un hydrofluoroalcane et au moins un alcool et/ou un hydrocarbure insaturé à titre de stabilisant, qui se caractérisent en ce que l'alcool est sélectionné parmi le méthanol, l'éthanol et l'éthylène glycol, en une quantité pondérale au moins égale à environ 0,001 % et inférieure à 0,1 % du poids de l'hydrofluoroalcane et l'hydrocarbure insaturé parmi les alcènes en C5-C6, en une quantité pondérale au moins égale à environ 0,001 % et inférieure à environ 0,5 % du poids de l'hydrofluoroalcane. De préférence, l'alcool utilisé comme stabilisant est présent en une quantité supérieure ou égale à 0,005 % et inférieure ou égale à environ 0,09 % du poids d'hydrofluoroalcane. De préférence, l'hydrocarbure insaturé utilisé comme stabilisant est présent en une quantité d'environ 0,005 % à environ 0,25 % du poids de l'hydrofluoroalcane.

L'alcool est avantageusement choisi parmi le méthanol et l'éthylène glycol, le méthanol étant tout particulièrement préféré. L'hydrocarbure insaturé est avantageusement choisi parmi le 2-méthylbut-2-ène, le 2-méthylbut-1-ène, le 3-méthylbut-1-ène et leurs mélanges.

Dans les compositions selon l'invention, l'hydrofluoroalcane peut être tout hydrofluoroalcane tel que décrit plus haut. Il est avantageusement le 1,1-dichloro-1-fluoroéthane.

Selon une forme de réalisation préférée de l'invention, les compositions comprennent, en plus de l'hydrofluoroalcane, conjointement au moins un alcool et au moins un hydrocarbure insaturé.

En variante, les compositions selon l'invention peuvent également contenir d'autres additifs accompagnant habituellement les hydrofluoroalcanes. Elles peuvent également contenir d'autres stabilisants.

Les exemples suivants illustrent l'invention. Les exemples 1 à 4, 9, 15, 19 et 20, annotés R sont donnés à titre de comparaison. Les exemples 5 à 8, 10 à 14, 16 à 18 et 21 à 23 sont réalisés selon l'invention.

### Exemples 1-8

Du 1,1-dichloro-1-fluoroéthane a été conservé à 50 °C dans des bonbonnes en inox, en présence de quantités variables de divers stabilisants et de FeCl₃. Les principales impuretés organiques présentes dans le 1-1-dichloro-1-fluoroéthane initialement, puis après 7 jours de traitement, ont été analysées par chromatographie en phase gazeuse. Ces impuretés sont essentiellement le 1-chloro-1-fluoroéthylène (VCF), le 1,1-dichloroéthylène (VC2), le 1-chloro-1,1-difluoroéthane (HCFC-142b) et le 1,1,1-trichloroéthane (T111). On a également mesuré la teneur en ions fluorures. Les résultats sont consignés dans le tableau I ci-après.

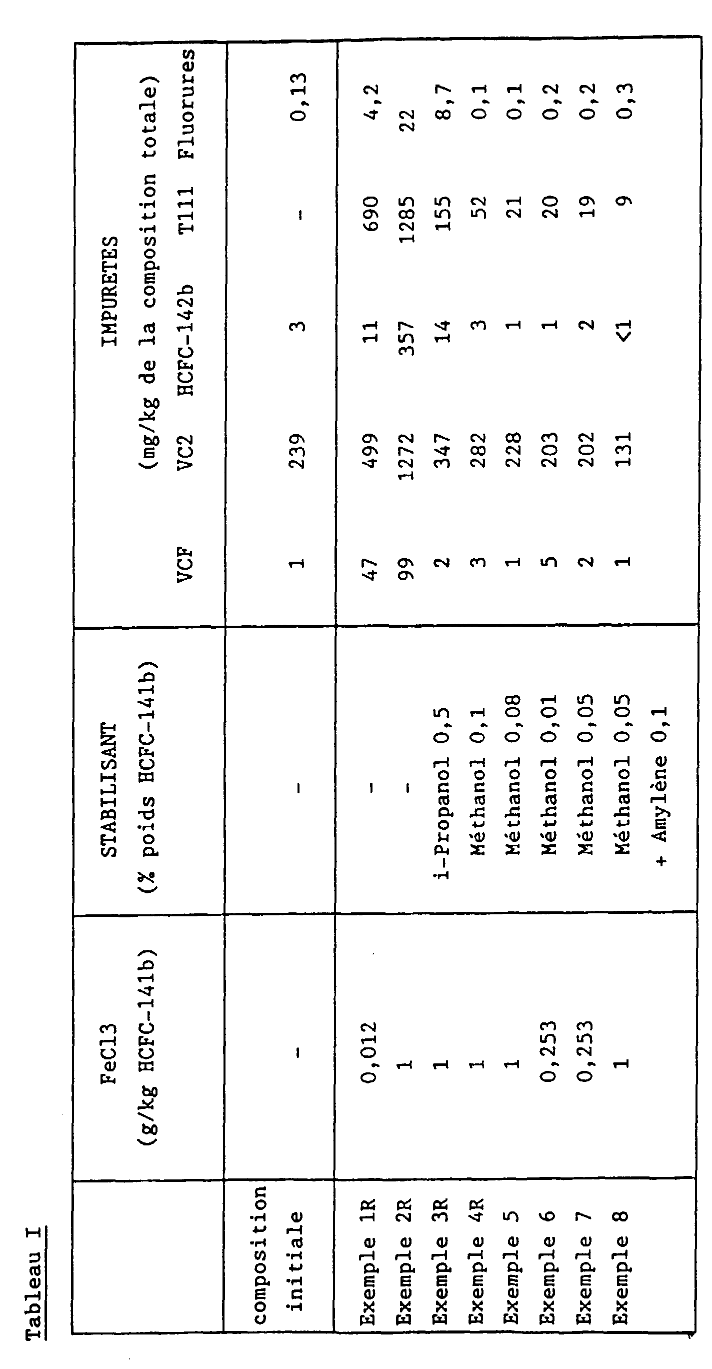

### Exemples 9-18

Du 1,1-dichloro-1-fluoroéthane de meilleure pureté que celui utilisé dans les exemples 1 à 8 a été conservé pendant 7 jours à 50°C dans des bonbonnes en inox, en présence de 1 gramme de FeCl₃ par kilo de 1,1-dichloro-1-fluoroéthane et de quantités variables de divers stabilisants.

Les résultats des analyses sont consignés dans le Tableau II ci-après.

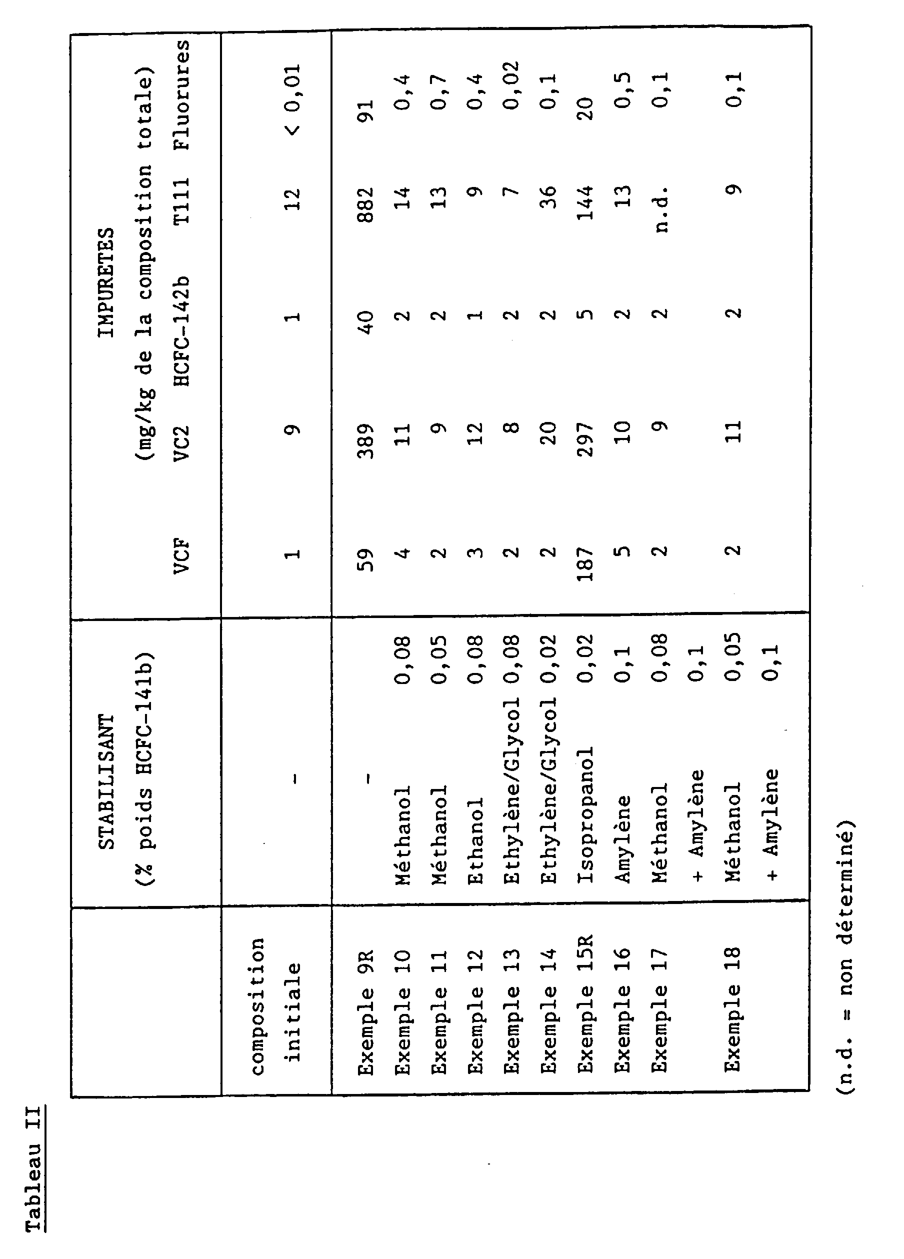

### Exemples 19-23

On fait bouillir à reflux du 1-1-dichloro-1-fluoroéthane de même pureté que celui utilisé pour les exemples 1 à 8, auquel on a ajouté divers stabilisants, dans un ballon bouilleur surmonté d'un extracteur Soxhlet et d'un réfrigérant, en présence d'éprouvettes métalliques placées l'une dans le bouilleur, une deuxième dans l'extracteur Soxhlet et une troisième dans le réfrigérant. Les éprouvettes métalliques en acier Thomas développent globalement une surface de 832 mm². L'agressivité des compositions stabilisées de 1-1-dichloro-1-fluoroéthane est évaluée par mesure de la perte en poids des éprouvettes, après 7 jours. Les résultats sont rassemblés au tableau III.

**Tableau III**

| | Ex.19R | Ex.20R | Ex.21 | Ex.22 | Ex.23 |
|---|---|---|---|---|---|
| Stabilisant (% poids par rapport au HCFC-141b) | | | | | |
| Méthanol | 0,5% | 0,1% | 0,05% | 0,01% | 0,05% |
| Amylène | - | - | - | - | 0,01% |

| Pertes en poids (g/m².jour) | | | | | |
|---|---|---|---|---|---|
| Ballon | 0,05 | 0 | 0,02 | 0,02 | 0,02 |
| Soxhlet | 0,32 | 0,36 | 0,02 | 0,03 | 0 |
| Réfrigérant | 0,17 | 0,02 | 0 | 0,05 | 0 |
| perte totale | 0,54 | 0,38 | 0,04 | 0,10 | 0,02 |

## Revendications

1. Procédé de stabilisation d'un hydrofluoroalcane acyclique ou alicyclique, de formule générale CₐH_{b}F_{c}Cl_{d} dans laquelle a est un nombre entier de 1 à 6, b est un nombre entier de 1 à 13, c est un nombre entier de 1 à 13 et d est un nombre entier de 0 à 8, avec b + c + d = 2 a + 2 lorsque le composé est acyclique et avec b + c + d = 2a lorsque le composé est alicyclique, à l'encontre de la dégradation provoquée par les acides de Lewis de type halogénures métalliques, selon lequel on ajoute audit hydrofluoroalcane au moins un alcool et/ou au moins un hydrocarbure insaturé à titre de stabilisant, caractérisé en ce qu'on sélectionne l'alcool parmi le méthanol, l'éthanol et l'éthylène glycol et l'hydrocarbure insaturé parmi les alcènes en C5-C6, et en ce qu'on met en oeuvre ledit alcool en une quantité pondérale au moins égale à environ 0,001 % et inférieure à 0,1 % du poids de l'hydrofluoroalcane et ledit hydrocarbure insaturé en une quantité au moins égale à environ 0,001 % et inférieure à environ 0,5 % du poids de l'hydrofluoroalcane.

2. Procédé selon la revendication 1 caractérisé en ce que l'alcool sélectionné est le méthanol.

3. Procédé selon la revendication 1 caractérisé en ce qu'on ajoute l'alcool en une quantité supérieure ou égale à environ 0,005 % et inférieure ou égale à environ 0,09 % du poids d'hydrofluoroalcane.

4. Procédé selon la revendication 1 caractérisé en ce qu'on sélectionne l'hydrocarbure insaturé parmi le 2-méthylbut-2-ène, le 2-méthylbut-1-ène, le 3-méthylbut-1-ène et leurs mélanges.

5. Procédé selon la revendication 1 caractérisé en ce qu'on ajoute au moins un alcool et au moins un hydrocarbure insaturé.

6. Procédé selon la revendication 1 caractérisé en ce qu'il est appliqué à la stabilisation du 1,1-dichloro-1-fluoroéthane.

7. Composition stabilisée à l'encontre de la dégradation provoquée par les acides de Lewis de type halogénures métalliques, comprenant au moins un hydrofluoroalcane acyclique ou alicyclique, de formule générale CₐH_{b}F_{c}Cl_{d} dans laquelle a est un nombre entier de 1 à 6, b est un nombre entier de 1 à 13, c est un nombre entier de 1 à 13 et d est un nombre entier de 0 à 8, avec b + c + d = 2 a + 2 lorsque le composé est acyclique et avec b + c + d = 2a lorsque le composé est alicyclique, et au moins un alcool et/ou au moins un hydrocarbure insaturé à titre de stabilisant caractérisé en ce que l'alcool est sélectionné parmi le méthanol, l'éthanol et l'éthylène glycol, en une quantité pondérale au moins égale à environ 0,001 % et inférieure à 0,1 % du poids de l'hydrofluoroalcane et l'hydrocarbure insaturé parmi les alcènes en C5-C6, en une quantité pondérale au moins égale à environ 0,001 % et inférieure à environ 0,5 % du poids de l'hydrofluoroalcane.

8. Composition selon la revendication 7, caractérisée en ce que l'alcool est le méthanol.

9. Composition selon la revendication 7 caractérisée en ce que l'alcool est présent en une quantité supérieure ou égale à 0,005 % et inférieure ou égale à environ 0,09 % du poids d'hydrofluoroalcane.

10. Composition selon la revendication 7 caractérisée en ce que l'hydrocarbure insaturé est choisi parmi le 2-méthylbut-2-ène, le 2-méthylbut-1-ène, le 3-méthylbut-1-ène et leurs mélanges.

11. Composition selon la revendication 7 caractérisée en ce qu'elle contient au moins un alcool et au moins un hydrocarbure insaturé.

12. Composition selon la revendication 7, caractérisée en ce que l'hydrofluoroalcane comprend du 1,1-dichloro-1-fluoroéthane.

## Patentansprüche

1. Verfahren zur Stabilisierung eines acyclischen oder alicyclischen Hydrofluoralkans der allgemeinen Formel CₐH_{b}F_{c}Cl_{d}, in der a eine ganze Zahl von 1 bis 6 ist, b eine ganze Zahl von 1 bis 13 ist, c eine ganze Zahl von 1 bis 13 ist und d eine ganze Zahl von 0 bis 8 ist, mit b + c + d = 2 a + 2, wenn die Verbindung acyclisch ist, und mit b + c + d = 2 a, wenn die Verbindung alicyclisch ist, gegen den durch die Lewis-Säuren vom Metallhalogenid-Typ verursachten Abbau, gemäß welchem Verfahren man besagtem Hydrofluoralkan wenigstens einen Alkohol und/oder wenigstens einen ungesättigten Kohlenwasserstoff als Stabilisator hinzufügt, dadurch gekennzeichnet, daß man den Alkohol unter Methanol, Ethanol und Ethylenglykol und den ungesättigten Kohlenwasserstoff unter den C₅-C₆-Alkenen auswählt, und dadurch, daß man besagten Alkohol in einer Gewichtsmenge von wenigstens gleich etwa 0,001% und kleiner 0,1% des Hydrofluoralkangewichts und besagten ungesättigten Kohlenwasserstoff in einer Menge von wenigstens gleich etwa 0,001% und kleiner etwa 0,5% des Hydrofluoralkangewichts verwendet.

2. Verfahren gemäß Anspruch 1, dadurch gekennzeichnet, daß der ausgewählte Alkohol Methanol ist.

3. Verfahren gemäß Anspruch 1, dadurch gekennzeichnet, daß man den Alkohol in einer Menge zufügt, die größer oder gleich etwa 0,005% und kleiner oder gleich etwa 0,09% des Hydrofluoralkangewichts ist.

4. Verfahren gemäß Anspruch 1, dadurch gekennzeichnet, daß man den ungesättigten Kohlenwasserstoff unter 2-Methylbut-2-en, 2-Methylbut-1-en, 3-Methylbut-1-en und deren Gemischen auswählt.

5. Verfahren gemäß Anspruch 1, dadurch gekennzeichnet, daß man wenigstens einen Alkohol und wenigstens einen ungesättigten Kohlenwasserstoff zufügt.

6. Verfahren gemäß Anspruch 1, dadurch gekennzeichnet, daß es auf die Stabilisierung von 1,1-Dichlor-1-fluorethan angewendet wird.

7. Gegen den durch die Lewis-Säuren vom Metallhalogenid-Typ verursachten Abbau stabilisierte Zusammensetzung, die wenigstens ein acyclisches oder alicyclisches Hydrofluoralkan der allgemeinen Formel CₐH_{b}F_{c}Cl_{d}, in der a eine ganze Zahl von 1 bis 6 ist, b eine ganze Zahl von 1 bis 13 ist, c eine ganze Zahl von 1 bis 13 ist und d eine ganze Zahl von 0 bis 8 ist, mit b + c + d = 2 a + 2, wenn die Verbindung acyclisch ist, und mit b + c + d = 2 a, wenn die Verbindung alicyclisch ist, und wenigstens einen Alkohol und/oder wenigstens einen ungesättigten Kohlenwasserstoff als Stabilisator umfaßt, dadurch gekennzeichnet, daß der Alkohol unter Methanol, Ethanol und Ethylenglykol in einer Gewichtsmenge von wenigstens gleich etwa 0,001% und kleiner 0,1% des Hydrofluoralkangewichts und der ungesättigte Kohlenwasserstoff unter den C₅-C₆-Alkenen in einer Gewichtsmenge von wenigstens gleich etwa 0,001% und kleiner etwa 0,5% des Hydrofluoralkangewichts ausgewahlt ist.

8. Zusammensetzung gemäß Anspruch 7, dadurch gekennzeichnet, daß der Alkohol Methanol ist.

9. Zusammensetzung gemäß Anspruch 7, dadurch gekennzeichnet, daß der Alkohol in einer Menge von größer oder gleich 0,005% und kleiner oder gleich etwa 0,09% des Hydrofluoralkangewichts vorhanden ist.

10. Zusammensetzung gemäß Anspruch 7, dadurch gekennzeichnet, daß der ungesättigte Kohlenwasserstoff unter 2-Methylbut-2-en, 2-Methylbut-1-en, 3-Methyl-but-1-en und deren Gemischen ausgewählt ist.

11. Zusammensetzung gemäß Anspruch 7, dadurch gekennzeichnet, daß sie wenigstens einen Alkohol und wenigstens einen ungesättigten Kohlenwasserstoff enthält.

12. Zusammensetzung gemäß Anspruch 7, dadurch gekennzeichnet, daß das Hydrofluoralkan 1,1-Dichlor-1-fluorethan umfaßt.

## Claims

1. Process for stabilising an acyclic or alicyclic hydrofluoroalkanes of general formula C₃H_{b}F_{c}Cl_{d} in which a is an integer from 1 to 6, b is an integer from 1 to 13, c is an integer from 1 to 13 and d is an integer from 0 to 8, with b + c + d = 2a + 2 when the compound is acyclic and with b + c + d = 2a when the compound is alicyclic against degradation caused by Lewis acids of metal halide type, according to which at least one alcohol and/or at least one unsaturated hydrocarbon are added as stabiliser to the said hydrofluoroalkane, characterised in that the alcohol is chosen from methanol, ethanol and ethylene glycol and the unsaturated hydrocarbon from C₅-C₆ alkenes, and in that the said alcohol is used in a quantity by weight of at least approximately 0.001 % and less than 0.1 % of the weight of the hydrofluoroalkane and the said unsaturated hydrocarbon in a quantity of at least approximately 0.001 % and less than approximately 0.5 % of the weight of the hydrofluoroalkane.

2. Process according to Claim 1, characterised in that the chosen alcohol is methanol.

3. Process according to Claim 1, characterised in that the alcohol is added in a quantity greater than or equal to approximately 0.005 % and less than or equal to approximately 0.09 % of the weight of hydrofluoroalkane.

4. Process according to Claim 1, characterised in that the unsaturated hydrocarbon is chosen from 2-methyl-2-butene, 2-methyl-1-butene, 3-methyl-1-butene and mixtures thereof.

5. Process according to Claim 1, characterised in that at least one alcohol and at least one unsaturated hydrocarbon are added.

6. Process according to Claim 1, characterised in that it is applied to the stabilisation of 1,1-dichloro-1-fluoroethane.

7. Composition stabilised against degradation caused by Lewis acids of metal halide type, comprising at least one acyclic or alicyclic hydrofluoroalkanes of general formula CₐH_{b}F_{c}Cl_{d} in which a is an integer from 1 to 6, b is an integer from 1 to 13, c is an integer from 1 to 13 and d is an integer from 0 to 8, with b + c + d = 2a + 2 when the compound is acyclic and with b + c + d = 2a when the compound is alicyclic, and at least one alcohol and/or at least one unsaturated hydrocarbon as stabiliser, characterised in that the alcohol is chosen from methanol, ethanol and ethylene glycol, in a quantity by weight of at least approximately 0.001 % and less than 0.1 % of the weight of the hydrofluoroalkane and the unsaturated hydrocarbon from C₅-C₆ alkenes, in a quantity by weight of at least approximately 0.001 % and less than approximately 0.5 % of the weight of the hydrofluoroalkane.

8. Composition according to Claim 7, characterised in that the alcohol is methanol.

9. Composition according to Claim 7, characterised in that the alcohol is present in a quantity greater than or equal to 0.005 % and less than or equal to approximately 0.09 % of the weight of hydrofluoroalkane.

10. Composition according to Claim 7, characterised in that the unsaturated hydrocarbon is chosen from 2-methyl-2-butene, 2-methyl-1-butene, 3-methyl-1-butene and mixtures thereof.

11. Composition according to Claim 7, characterised in that it contains at least one alcohol and at least one unsaturated hydrocarbon.

12. Composition according to Claim 7, characterised in that the hydrofluoroalkane includes 1,1-dichloro-1-fluoroethane.
